# EUROPÄISCHE PATENTSCHRIFT

(11) **EP 0 429 971 B1**
(45) Veröffentlichungstag und Bekanntmachung des Hinweises auf die Patenterteilung: **25.09.1996**
(21) Anmeldenummer: 90121859.4
(22) Anmeldetag: 15.11.1990
(51) Int. Cl.: G10K 15/00, G11B 20/00

(54) **Verfahren und Vorrichtung zur Herstellung subliminaler Tonaufnahmen**
Method and apparatus to produce subliminal sound recording
Procédé et dispositif pour faire de l'enregistrement de son subliminal

(30) Priorität: 22.11.1989 US 440244
(43) Veröffentlichungstag der Anmeldung: 05.06.1991
(73) Patentinhaber: Taylor, Eldon, Las Vegas, Nevada 89119 (US)
(72) Erfinder: Taylor, Eldon, Las Vegas, Nevada 89119 (US); Woodhams, James Ray, Henderson, Nevada 89014 (US)
(74) Vertreter: Munk, Ludwig, Dipl.-Ing.

(56) Entgegenhaltungen:
- DE-U- 8 911 253
- US-A- 4 777 529

## Beschreibung

Die Erfindung betrifft gemäß einem ersten Erfindungsgedanken ein Verfahren zur Herstellung von subliminalen Tonaufnahmen und gemäß einem weiteren Erfindungsgedanken eine Vorrichtung zur Durchführung des Verfahrens.

Es ist eine wohlbekannte Tatsache, daß jeder Mensch zwei Gehirne hat, umgangssprachlich das rechte und linke Gehirn genannt, die in Wirklichkeit die rechte und linke Hemisphäre des menschlichen Gehirns sind. Bei der überwiegenden Mehrheit der Menschen ist die linke Gehirnhemisphäre verantwortlich für Dinge wie mathematische und linguistische Fähigkeiten, während die rechte Gehirnhemisphäre das kreative und emotionale Zentrum ist. Die linke Hemisphäre ist das Zentrum von Logik und Vernunft mit Abwehrmechanismen wie Rationalisieren, die sich auf Logik und Vernunft aufbauen. Die rechte Hemisphäre ist im Gegensatz dazu kritikunfähig. Die meisten Forscher ordnen Logik und bewußten Verstand der linken Hemisphäre und emotionelle und unterbewußte Prozesse der rechten Hemisphäre zu.

Man nimmt an, daß die linke Hemisphäre sich für nüchterne Korrektheit interessiert, während die rechte Hemisphäre mehr an übergreifenden Assoziationen und Beziehungen interessiert ist. Man nimmt an, daß die linke Hemisphäre Sprache nach Buchstaben und gemäß den Sprachregeln betrachtet, während die rechte Hemisphäre Sprache räumlich und emotional betrachtet, wobei sie die Wörter in einem Verfahren, das als "unterbewußte Celebration" bezeichnet wird, umdreht und sie sogar genauso hört, wie unsere Augen die Welt sehen ... umgekehrt.

Es wurde vorgeschlagen, die Funktionsweise des Gehirns zu nutzen, um subliminale Nachrichten dorthin zu senden. Diese Nachrichten scheinen direkt die Emotionen anzusprechen und bewirken, daß das Verhalten die Vernunft übergeht. Subliminale Nachrichten können entweder visuell oder akustisch gesendet werden.

Im Fall der audiosubliminalen Darbietung, unter Verwendung von Tonbändern, kann eine subliminale Nachricht als verbaler Reiz definiert werden, der unterhalb der Bewußtheitsschwelle des Zuhörers wahrgenommen wird. Wahrnehmung bezieht sich hierbei auf den Prozeß, bei dem Reize auf irgendeinem Level unterhalb der Bewußtheitsschwelle (Aufmerksamkeitsschwelle) erkannt und registriert werden. Damit bei einem Individuum der Prozeß der Wahrnehmung ablaufen kann, muß der Reiz ausreichen, um eine Neuronenaktivität im Gehirn auszulösen. Hier liegt ein Unterschied zu einer Situation, in der die Nachricht nicht nur unter der Schwelle der Bewußtheit liegt, sondern auch nicht wahrgenommen wird, wie z.B. ein Flüstern zwei Häuserblocks vom Hörer entfernt.

Bei der audiosubliminalen Darbietung werden gesprochene Worte unterhalb eines Primärträgers eingebracht. Der Hörer hört die gesprochenen Worte nicht bewußt, da sie in "weißem" Rauschen wie beispielsweise Klänge von Wind oder Wasser verborgen, oder psychoakustisch in oder unter die Musik eingearbeitet sind. Nichtsdestoweniger, wenn das Verfahren richtig durchgeführt wird, und die Lautstärke der Worte nicht nur im Verhältnis zur Lautstärke des Primärträgers wesentlich abgesenkt wird, wird der Reiz der gesprochenen Worte vom Hörer registriert.

Man kann sich als Analogie hierzu vorstellen, daß sich der verbale Reiz unter den Wellen von Naturgeräuschen und Musik bewegt, wie sich ein U-Boot unter den Wellen an der Oberfläche des Meeres bewegt. Das Außenrohr nimmt den Klang auf (sowohl die Oberflächenwellen als auch alles darunterliegende), sowie sie in den Gehörgang gelangen. Vom Gehörgang werden die Wellen auf das Trommelfell, oder das Mittelohr übertragen, wo die Vibrationen mittels Luftdruck und drei kleinen Knochen ins Innenohr weitergeleitet werden. Im Innenohr liegen die Cochleen, oder Schnecken, die mit sensorischen Zellen ausgestattet sind, die den Klang aufnehmen und dem Gehirn die aus dem Klang entstehenden Impulse zuleiten. Die Reize lösen schließlich im Gehirn eine Neuronenaktivität aus. Millionen von Neuronen tragen Nachrichteneinheiten, die durch Reize ausgelöst wurden, über die Synapsen im Gehirn und jede dieser Nachrichten wirbt gleichzeitig um bewußte Aufmerksamkeit.

Neuronen haben keinen neutralen Status; sie sind entweder an oder aus. Die Schwelle der Bewußtheit bzw. der "Wahrnehmungslevel", der existiert und unterhalb dieser Schwelle arbeitet, ist nichts anderes als eine neurale Erregung. Ohne neurale Erregung kann es keine Wahrnehmung geben. Untersuchungen der Gehirnwellenaktivität haben eine erhöhte Schemenaktivität bei Testpersonen bestätigt, die Musik mit darin enthaltenen subliminalen Nachrichten hörten, im Vergleich zu Testpersonen, die die gleiche Musik ohne die subliminalen Nachrichten hörten.

Die Nuancen innerhalb audiosubliminaler Darbietungsformen variieren sehr stark. Ein Verfahren beinhaltet eine "Zeitkompression" der Verbalnachricht, wobei entweder die menschliche Stimme bis zu einem hochfrequenten Quietschen beschleunigt wird, oder die Worte digital komprimiert werden, was ein Entfernen der Zwischenräume zwischen den Klängen beinhaltet und in einem einzigen andauernden Geräusch resultiert.

Eine andere Methode ist als "Multifrequente Aussagen" bekannt und beinhaltet die Verwendung von einzelnen Aussagen, die auf verschiedenen Frequenzen übereinandergelegt und dann gleichzeitig wiedergegeben werden. Meist werden diese multifrequenten Nachrichten in Frequenzbereichen aufgenommen, die ein herkömmlicher Kassettenrekorder nicht wiedergeben kann. Audiologen sind der Ansicht, daß solche multifrequenten Nachrichten für den Menschen nicht wahrnehmbar sind.

Im Gegensatz zu diesen zwei exotischen Methoden subliminaler Nachrichtenübertragung gibt es eine Anzahl bekannter und bewiesener Verfahren für die Übertragung verbaler, subliminaler Reize. Eine ist das Einbetten unkomplizierter Nachrichten. Eine andere ist als Back-Masking oder Metakontrast bekannt. Eine dritte benützt beides gleichzeitig.

Bei der Methode zum Senden subliminaler Nachrichten, die als Metakontrast oder Back-Masking bekannt ist, werden die subliminalen Nachrichten rückwärts aufgenommen. Wenn subliminale Nachrichten umgedreht oder rückwärts abgespielt an das Gehirn geschickt werden, scheinen diese subliminalen Nachrichten emotionelle Ausdrücke und Reaktionen auszulösen, die in ihrem Ursprung oft als rechtshemisphärisch angesehen werden. Es wird vermutet, daß Hardrockaufnahmen diesen Prozeß dazu benutzt haben, um satanische, drogen- oder sexbezogene Nachrichten an das Gehirn des Zuhörers zu senden.

Der erste subliminale Spurenmischer wurde offensichtlich in den Siebziger Jahren von Prof. Hal Becker von der Tulane University entwickelt. Becker's Gerät, einfach "Black Box" genannt, wurde eingesetzt, um gesprochene Worte mit Musik zu mischen. Becker's Gerät war monophon und wurde 1979 in einem Supermarkt in New Orleans getestet. Die Black Box mischte die Worte "Du sollst nicht stehlen" und "Ehrlich währt am längsten" in das Musikübertragungssystem des gesamten Ladens. Dieser Test erbrachte Berichten zufolge eine substantielle Verminderung von Geldknappheit, Inventarbeschädigung und Ladendiebstählen.

Das elektronische Mischen von Musik (Primärträger) und verbaler Nachricht (die subliminale Nachricht) hilft, ein konstantes Signaldifferntial zwischen den beiden zu gewährleisten. man nimmt an, daß das Signaldifferntial ausschlaggebend für die Effektivität ist, mit der das Gehirn den subliminalen Prozeß registriert.

Die US-A 4 777 529 offenbart ein Verfahren zur Herstellung von subliminalen Tonaufnahmen mit folgenden Schritten:
a) die Selektion eines Klanges, der als Primärträger verwendet wird,
b) die Selektion der Sprache, die unterhalb des Primärträgers als subliminale Nachricht eingefügt wird
e) das Mischen des Primärträgerklanges mit der Sprache der subliminalen Nachricht so, daß der Schalldruckpegel der subliminalen Nachricht um einen konstanten Dezibelabstand unterhalb dem des Primärträgerklanges bleibt,
f) die Aufnahme der Mischung aus Primärträgerklang und Sprache der subliminalen Nachricht, wobei eine subliminale Tonaufnahme entsteht.

Die US-A 4 777 529 offenbart überdies eine Vorrichtung zur Durchführung dieses Verfahrens mit
a) Hilfsmitteln zum Abspielen eines Klanges, der als Primärträger verwendet werden soll,
b) Hilfsmittel zum Abspielen der Sprache, die unterhalb des Primärträgers als subliminale Nachricht eingefügt werden soll,
c) Hilfsmittel zum Mischen des Primärträgerklanges mit der Sprache der subliminalen Nachricht so daß der Schalldruckpegel der subliminalen Nachricht um einen konstanten Dezibelabstand unterhalb dem des Primärträgerklanges bleibt,
d) Hilfsmittel zur Aufnahme der Mischung aus Primärträgerklang und Sprache der subliminalen Nachricht, wobei eine subliminale Tonaufnahme entsteht.

Obiges Verfahren und obige Vorrichtung beziehen die aufgabenorientiert arbeitende linnke und rechte Gehirnhemisphäre nicht befriedigend entsprechend ihrer individuellen Eigenschaften ein.

Hauptaufgabe der vorliegenden Erfindung ist die Schaffung einer Methode zur Aufnahme audosubliminaler Bänder, mit der die subliminale Sprache so unter den primärträger eingefügt wird, daß sie regenerierbar ist, d.h. das Vorhandensein subliminaler Sprache auf dem Tonband mit elektronischen Mitteln nachweisbar ist, wobei durch die subliminale Sprache die linke und die rechte Gehirnhemispähre entsprechend ihrer individuellen Eigenschaften einbezogen werden sollen. Diese Aufgabe wird durch die Maßnahmen des Anspruchs 1 gelöst.

Außerdem ist es eine Aufgabe der vorliegenden Erfindung, eine Vorrichtung zur Aufnahme regenerierbarer, subliminaler Sprache unterhalb eines Primärträgers, unter Gewährleistung eine konstanten Pegeldifferntials zu schaffen, wobei durch die subliminale Sprache die linke und die rechte Gerhirnhemisphäre entsprechend ihrer individuellen Eigenschaften einbezogen werden sollen.

Diese Aufgabe wird durch die Maßnahmen des Anspruchs 9 gelöst.

Eine Eigenschaft der vorliegenden Erfindung ist, daß eine Vorrichtung geliefert wird, die subliminale Sprache unterhalb eines Primärträgers auf ein Tonband einfügt, wobei vorwärts und rückwärts maskierte (Metakontrast) Sprache kombiniert wird, die volle Echoverhallung und kanonartige Sprache gemeinsam mit verschiedenen Stimmencharakteristika enthält.

Die vorliegende Erfindung arbeitet mit der sogenannten "Whole-Brain" Methode. Bei der Whole-Brain Methode werden, zusätzlich zur Verwendung eines Primärträgers wie Musik, zwei Tonkanäle benutzt, um subliminale Nachrichten an das Gehirn zu liefern. Auf einem Kanal, mit Zugang zur linken Gehirnhemisphäre, besteht die gelieferte Nachricht aus bedeutungsvoll gesprochen oder gesungenen, vorwärts-maskierten, erlaubenden Affirmationen, die kanonartig von einer männlichen, einer weiblichen und einer Kinderstimme dargeboten werden. Untersuchungen haben gezeigt, daß einzelne Individuen günstiger auf entweder eine männliche, eine weibliche oder eine Kinderstimme reagieren, je nach der persönlichen Vorliebe des Hörers. Auf dem anderen Kanal, mit Zugang zur rechten Gehirnhälfte, werden anweisende Nachrichten mit denselben Stimmen rückwärts aufgenommen (Metakontrast). Da die Hemisphären aufgabenorientiert arbeiten, werden sowohl die linke, als auch die rechte Gehirnhemisphäre entsprechend ihrer individuellen Eigenschaften einbezogen.

Vorteilhaft können weitere Nuancen hinzugefügt werden. So können die drei Stimmen kanonartig mit voller Echo/Hall Effektierung aufgenommen werden. Studien der einzelnen Gehirnhemisphären beweisen, daß sowohl die linke, als auch die rechte Gehirnhemisphäre mit einer Fähigkeit zur Identifizierung gesungener Worte reagieren. Wenn die Worte gesprochen werden, gibt es hemisphärische Einschränkungen, die die Fähigkeit des Gehirns beeinträchtigen, die Worte voll zu identifizieren und zu verstehen. Darüberhinaus zeigen Schnellernmethoden einen Vorteil der kanonartigen Kommunikationsmethode. Die Tonspuren werden unter Verwendung eines speziellen Prozessors gemischt, der Tonfrequenz in elektrische Impulse umwandelt, und die subliminale Nachricht führt, um sie in Stereo mit dem Primärträger zu synchronisieren.

Der Prozessor, unter dem Namen PAR Prozessor bekannt, erhält ein konstantes Pegeldifferential zwischen dem Primärträger und der subliminalen Sprache und sorgt dafür, daß nichts von der kanonartig und mit voller Echo/Hall Effektierung aufgenommenen subliminalen Nachricht verlorengeht.

Es ist auch möglich, als Primärträger fortlaufende Musik ohne Pausen und große Bewegungsunterschiede zu verwenden, oder "weißes" oder "rosa" Rauschen, wie Meeresrauschen, mit der Musik zu kombinieren. Die Dauer der Pausen in der Musik müssen ziemlich kurz sein, damit die Wirkung der subliminalen Nachrichten nicht verändert wird.

Nachstehend wird ein Ausführungsbeispiel der Erfindung anhand der Zeichnung näher erläutert.

In der Zeichnung zeigen:
- Figuren 1a bis 1k: schematisch eines der Masteraufnahmegeräte, die im System der vorliegenden Erfindung verwendet werden,
- Figur 2: schematisch einen der automatischen Spurenmischer der vorliegenden Erfindung, der den Primärträger mit der subliminalen Sprache bei gleichbleibendem Pegeldifferential mischt,
- Figur 3: schematisch den Schaltungsaufbau der Zeitverzögerung, die im System der vorliegenden Erfindung verwender wird,
- Figur 4: schematisch die Schaltung für eine der dreifarbigen LED-Anzeigen, die im System der vorliegenden Erfindung verwendet werden und
- Figur 5: schematisch die Stromversorgung, die im System der vorliegenden Erfindung verwendet wird.

Der erste Schritt ist die Selektion sowohl des Primärträgers und der subliminalen Sprache, die kombiniert werden sollen, um eine audiosubliminale Aufnahme herzustellen.

In der bevorzugten Anwendungsform beinhaltet der Primärträger beruhigende Naturgeräusche, wie z.B. fortwährendes Meeresrauschen. Es wird auch bevorzugt reine Instrumentalmusik verwendet, oder die Kombination solcher Musik mit den Naturgeräuschen. Das Hauptkriterium ist, daß, wenn überhaupt, nur wenige Pausen im Primärträger vorhanden sind, da die Hauptfunktion des Primärträgers ist, die Sprache zu verstecken, die benutzt wird, um die subliminale Nachricht zu transportieren.

Ebenfalls in der bevorzugten Anwendungsform wird die subliminale Nachricht nach gewissen Kriterien ausgesucht. So werden z.B. vorzugsweise positive Affirmationen in kurzen, kompakten Aussagen verwendet. Wenn der Gegenstand der subliminalen Nachricht Selbstverbesserung ist, werden Aussagen wie "Ich tue mein Bestes" oder "Ich bin erfolgreich" verwendet. Wenn der Gegenstand der subliminalen Nachricht Vergebung ist, werden Aussagen wie "Ich vergebe mir selbst" oder "mir wird vergeben" oder "Ich vergeben allen anderen" verwendet.

Ebenfalls wird vorzugsweise jede dieser Aussagen mit drei verschiedenen Stimmtypen aufgenommen, so wie die Stimme eines Mannes, die einer Frau und die eines Kindes. In der bevorzugten Anwendung liegt die männliche Stimme allgemein im Frequenzbereich zwischen 540-560 Hz, die weibliche Stimme allgemein im Frequenzbereich zwischen 1000-1050 Hz und die Kinderstimme allgemein im Frequenzbereich zwischen 950-1030 Hz. Diese Stimmenkombination wird verwendet, weil die Hörer dazu tendieren, auf gewisse Stimmtypen günstiger zu reagieren als auf andere. Die Sprache kann entweder gesprochen, gesungen oder aus beidem kombiniert sein, da manche Leute besser auf gesprochene Worte reagieren, während andere besser auf Gesang reagieren.

Eine erste Aufnahme, gewöhnlich auf Band, wird vorbereitet, die die Worte enthält, die die subliminale Sprache ergeben werden. Jede Aussage wird zuerst mit der männlichen Stimme aufgenommen, gefolgt von derselben Aussage mit der weiblichen Stimme und schließlich mit der Kinderstimme. Diese selbe Aussage kann dann wiederholt werden oder unterschiedliche Aussagen können eingestreut werden, alle mit den drei verschiedenen Stimmen aufgenommen.

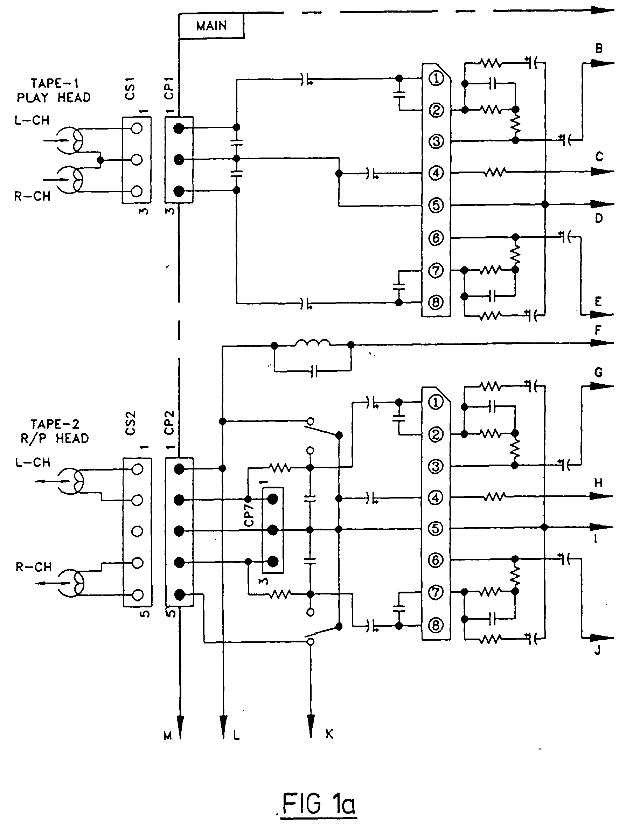

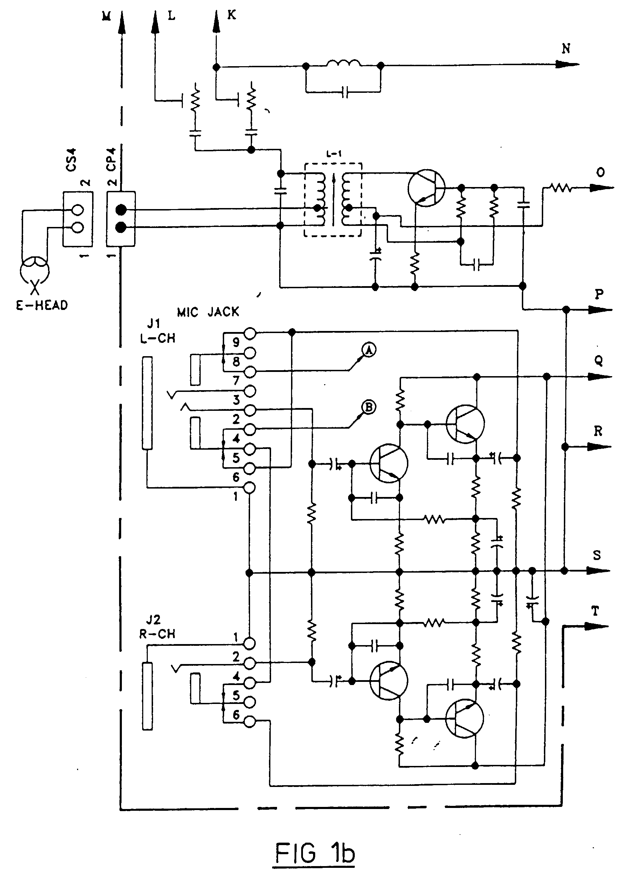

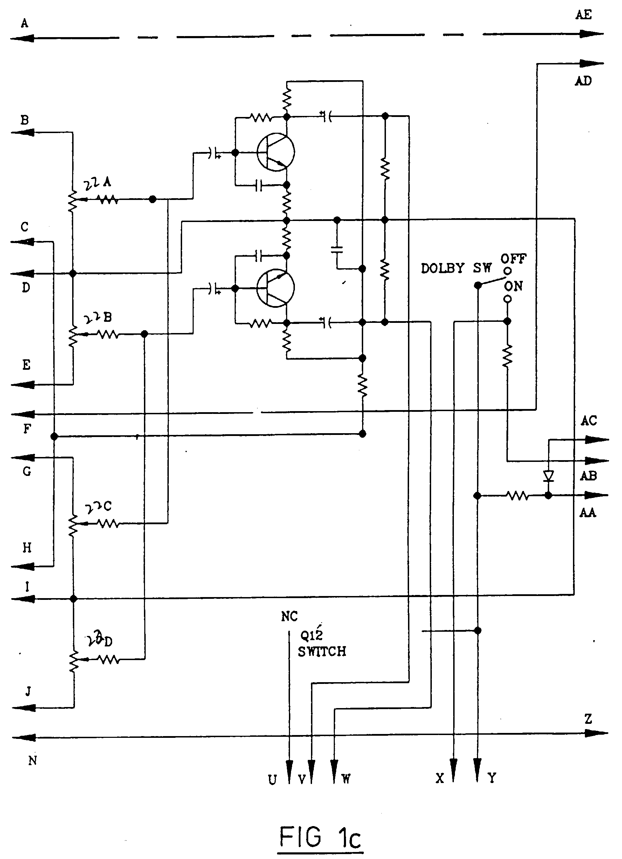

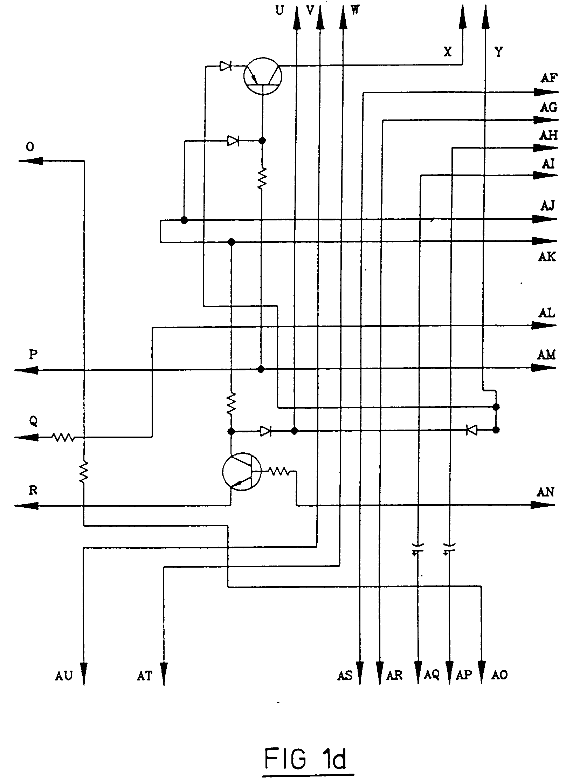

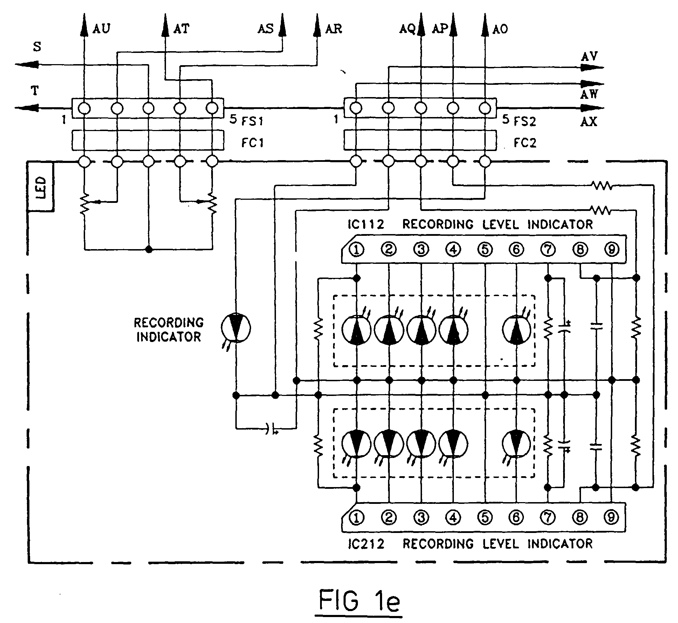

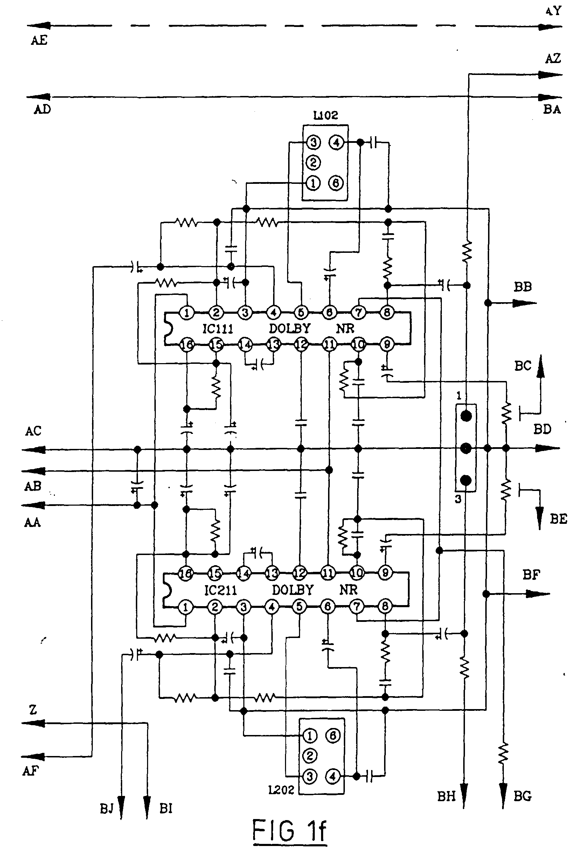

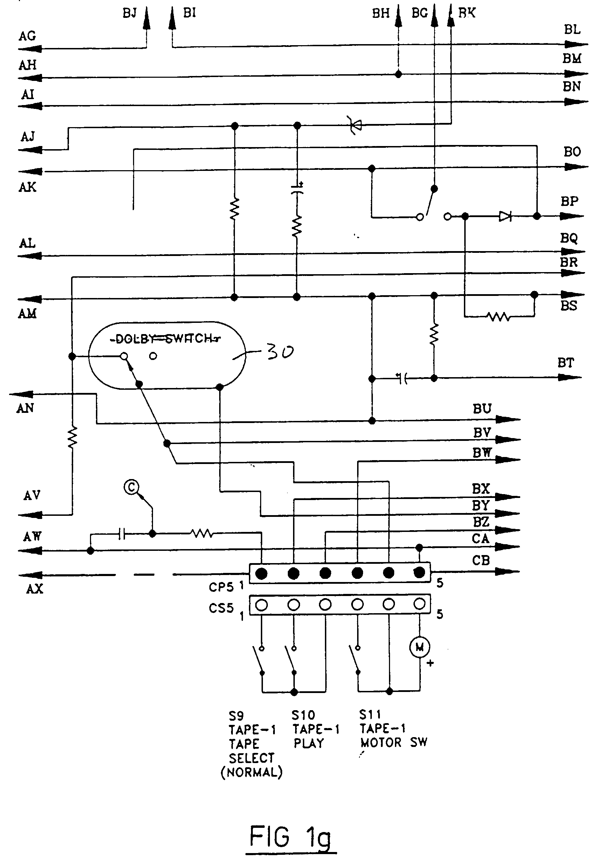

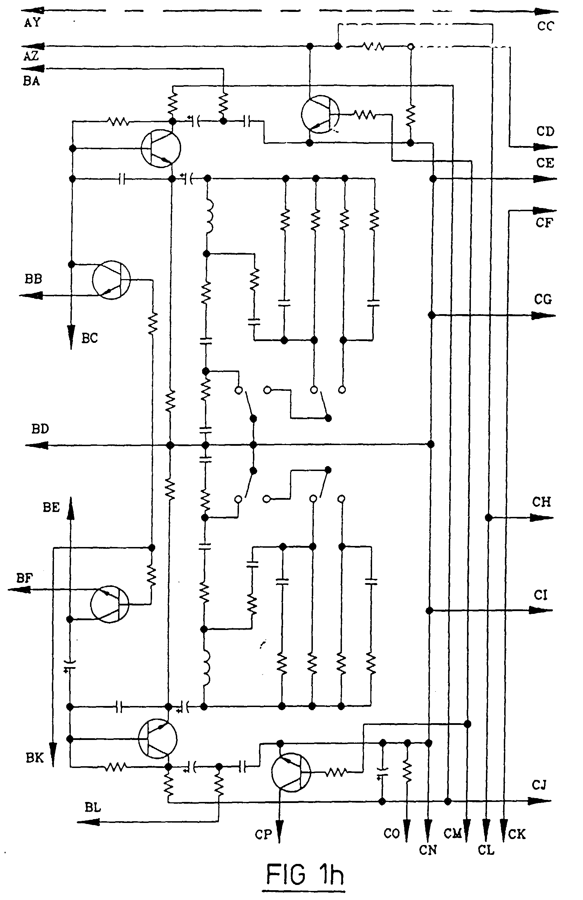

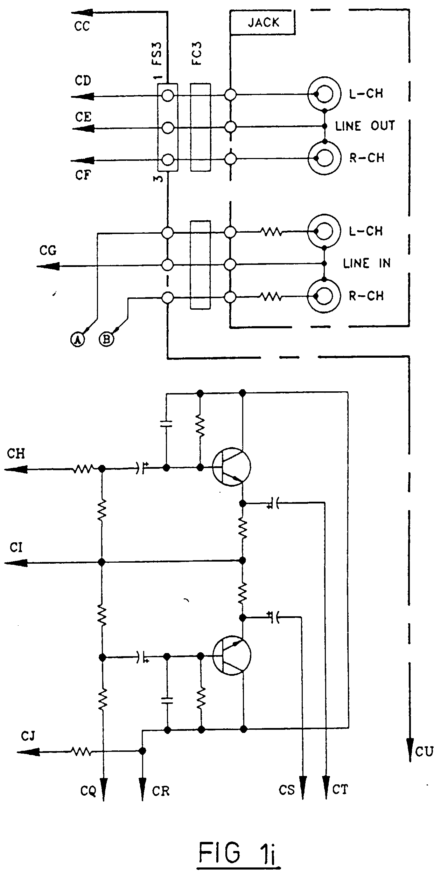

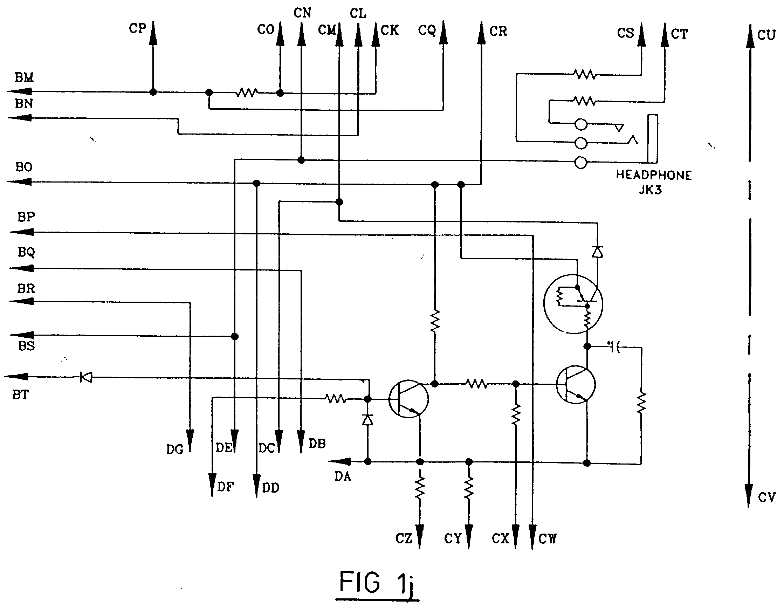

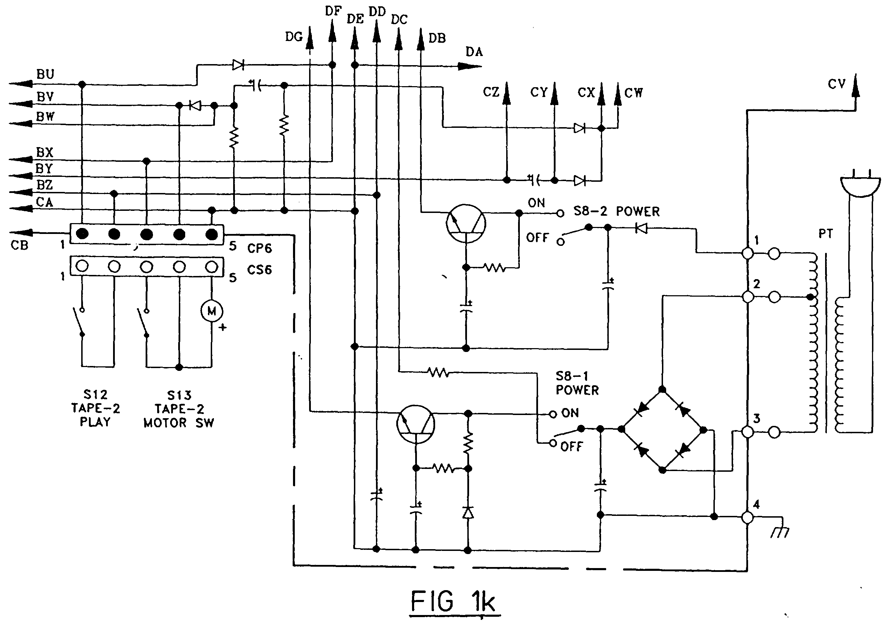

Die erste Sprache, die schließlich zur subliminalen Nachricht in der fertigen Aufnahme wird, wird in den Tonmischer eingespeist, der in Figur 1 dargestellt ist.

Im Mischer werden die Signale von zwei Wiedergabeköpfen auf dem ersten Wiedergabegerät abgespielt, das zum besseren Verständnis als Deck #2 bezeichnet wird, und zur weiteren Verarbeitung durch den Mischer geschickt. Jeder Wiedergabekopf ist mit einem vorher aufgenommenen Band geladen, das dieselbe Sprache enthält. Einer der Wiedergabeköpfe spielt die Sprache vorwärts ab, was dann beispielsweise der linke Kanal der fertigen Aufnahme wird und diese Sprache funktioniert dann als vorwärts-maskierte subliminale Sprache in der fertigen Aufnahme. Der andere Wiedergabekopf spielt die Sprache rückwärts ab, die in der fertigen Aufnahme den rechten Kanal ergibt, und diese Sprache wird rückwärts (oder in Metakontrast) zur weiteren Verarbeitung durch das System geschickt. Wenn diese Kanäle schließlich wieder zusammengefügt werden, beinhaltet die fertige Aufnahme die gleiche Nachricht sowohl vorwärts als auch rückwärts, aber auf unterschiedlichen Kanälen des Bandes.

Zusätzlich zu dieser kombinierten Vor- und Rückwärts-Mischung können drei einzelne Wiedergabegeräte verwendet werden - der Einfachheit halber hier als Deck #2, Deck #1 und Deck #3 bezeichnet. Deck #1 gibt dieselbe Sprache wieder wie Deck #2, aber mit einem Delay (Zeitverzögerung) von einer Sekunde. Deck #3 gibt dieselbe Sprache wieder wie Deck #2, aber mit einem Delay von zwei Sekunden. Alle drei Decks geben dieselbe Sprache sowohl vorwärts als auch rückwärts wieder. Wenn die Signale im Mischer zusammengefügt werden, ergibt sich aus der Ein- bzw. Zweisekundenverzögerung der drei Decks, die die Sprache wiedergeben, der Kanoneffekt.

Der linke Kanal der Sprache ist ganz im Vorwärts-Modus und der rechte Kanal der Sprache ganz im Rückwärts- oder Metakontrastmodus. Ob vorwärts oder rückwärts, jeder Kanal enthält dieselbe Nachricht im Kanoneffekt mit der Wiederholung in männlicher, weiblicher und Kinderstimme. Je nach Wunsch kann die volle Echo/Hall Effektierung elektronisch zu jedem Kanal hinzugefügt werden, um die Unterstützung der in der Sprache enthaltenen Nachricht zu verlängern.

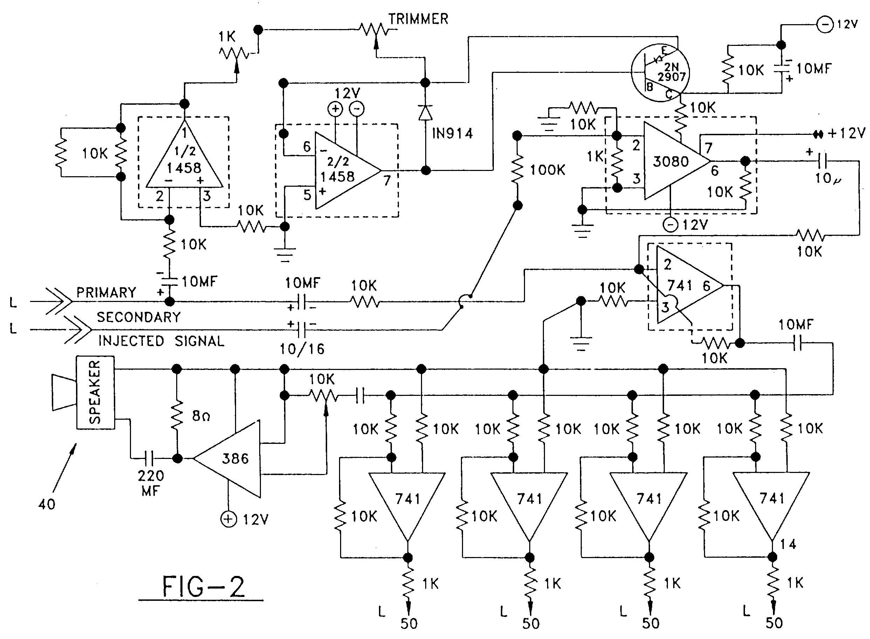

Diese subliminale Nachricht - linker Kanal im Vorwärtsmodus und rechter Kanal im Rückwärtsmodus - wird dann individuell mit dem Primärträger gemischt, um die Maskierung der subliminalen Nachricht zu erzielen. Das Mischen der subliminalen Nachricht mit dem Primärträger wird unter Verwendung der Schaltung durchgeführt, die schematisch in Figur 2 dargestellt ist.

Wie in Figur 2 dargestellt, wird erst der Signalpegel des Primärträgers getestet. Unter Verwendung eines variablen Widerstandes wird der Signalpegel des Primärträgers getestet und das Signal der subliminalen Nachricht, die in den Primärträger eingefügt werden soll wird so eingestellt oder verändert, daß ein konstantes Pegeldifferential zum Signalpegel des Primärträgers erreicht wird. Dieser Pegelunterschied ist vorzugsweise dergestalt, daß der Primärträger generell nicht mehr als 20 dB über dem Signalpegel der Sekundärsprache bzw. der subliminalen Nachricht liegt. In der am meisten bevorzugten Anwendung wird das Pegeldifferential so eingestellt, daß der Primärträger in etwa zwischen 3 dB und 5 dB über dem Signalpegel der Sekundärsprache oder dem Signal der subliminalen Nachricht liegt.

Das eingestellte Signal des Primärträgers und das Signal der subliminalen Nachricht werden dann kombiniert und durch einen geeigneten Verteiler verteilt, so wie Lautsprecher oder Verteiler-Vorverstärker zur endgültigen Aufzeichnung auf ein Tonbandgerät oder ein anderes geeignetes Aufzeichnungsgerät, wie in Figur 2 dargestellt.

Die Figuren 1a - 1k zeigen schematisch das Doppelkassettendeck, das im System der vorliegenden Erfindung verwendet wird. Es handelt sich hierbei um ein modifiziertes Doppelkassettendeck der Firma Tandy Corporation, National Parts Dept., Forth Worth, Texas 76101, das unter der Typenbezeichnung SCT-45-METAL im Handelt ist. Das Tandy Doppelkassettendeck ist ein herkömmliches Kassettendeck, das den Zwecken der vorliegenden Erfindung entsprechend modifiziert wurde.

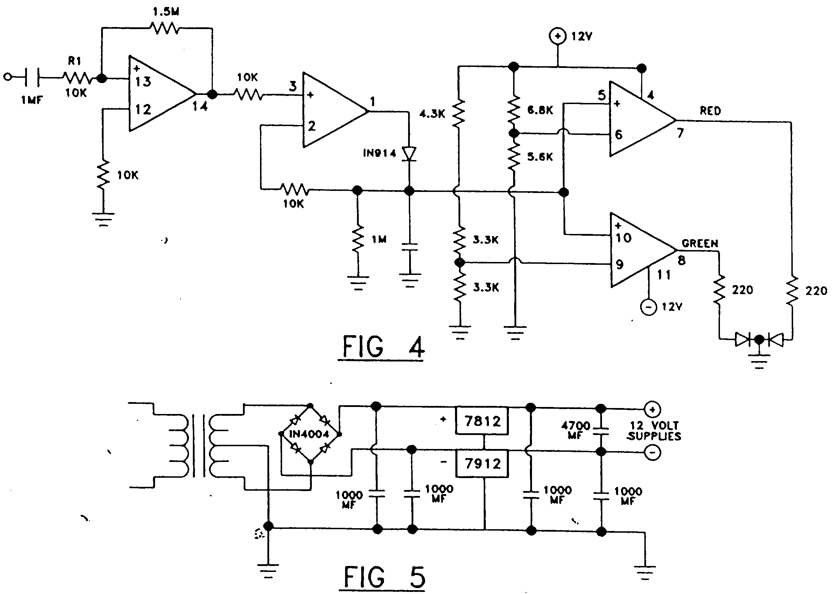

Die Bedienung des modifizierten Doppelkassettendecks der vorliegenden Erfindung wird nun beschrieben in Verbindung mit der Erklärung der Modifikationen, die an den Tandy Doppelkassettendeck vorgenommen worden sind. Ein voraufgenommenes Band, das die gewünschten subliminalen Nachrichten enthält, die als vorwärts maskierte Sekundärsignale unter das Primärsignal gelegt werden soll, wird in den Band-1 Wiedergabekopf eingelegt und ein voraufgenommenes Band, das als Sekundärsignal im Rückwärts- oder Metakontrastmodus unter das Primärsignal gelegt werden soll, wird in den Band-2 Wiedergabekopf eingelegt (siehe Figur 1a). Das voraufgenommene Band, das in den Band-2 Wiedergabekopf eingelegt wird, enthält die Nachricht ursprünglich im vorwärts-maskierten Modus, und eine der vorgenommenen Modifikationen ist das Umdrehen der Band-2 Wiedergabeköpfe, so daß das voraufgenommene Band in diesem Wiedergabekopf rückwärts abgespielt wird, um die Rückwärtsmaskierung, bzw. den Metakonstrast, herzustellen, der in die endgültige Subliminalaufnahme eingefügt wird. Das Signal von jedem Kanal des Band-1 Wiedergabekopfes und des Band-2 Wiedergabekopfes wird vorverstärkt und durch einen variablen Widerstand 22A, 22B, 22C, 22D geschickt, in dieser Reihenfolge zur individuellen Pegeljustierung. Die individuelle Pegeljustierung wird vom Benutzer unter Verwendung der LED-Anzeigeschaltung, die in Figur 4 dargestellt ist, manuell vorgenommen.

Nachdem jedes Signal auf den korrekten Pegel eingestellt worden ist, wobei es im bevorzugten Fall normalerweise nicht mehr als 1 dB Pegeldifferential zu den anderen Signalen, die von den anderen Wiedergabeköpfen im Kassettendeck kommen, aufweist, wird es durch ein Dolby Rauschunterdrückungssystem (Dolby NR) geschickt (Figur 1f) und dann zum Mischen mit dem Primärsignal vorverstärkt.

Eine weitere Modifikation, die an dem Tandy Doppelkassettendeck vorgenommen wurde ist, daß die Aufnahmefunktion des Decks außer Kraft gesetzt ist, da das Doppelkassettendeck in der vorliegenden Erfindung ausschließlich als Wiedergabegerät eingesetzt wird. Die Überspielfunktion ist aus demselben Grund ebenfalls außer Funktion gesetzt. Schließlich ist der Bandselektionsschalter modifiziert worden, um als Delayschaltung (Zeitverzögerung) verwendet werden zu können, wie unten ausgeführt wird.

Figur 2 zeigt schematisch den automatischen Spurenmischer der vorliegenden Erfindung, der das Primärträgersignal mit der subliminalen Sprache bei konstantem Pegeldifferential mischt. Wie oben besprochen, besteht das Primärträgersignal aus beruhigenden Naturgeräuschen, wie z.B. Meeresrauschen. Es wird ebenfalls vorzugsweise reine Instrumentalmusik oder die Kombination solcher Musik mit den Naturgeräuschen verwendet.

Wie Figur 2 zeigt, wird das Primärsignal zuerst kontinuierlich überprüft, um den Signalpegel zu bestimmen. Das Sekundärsignal wird kontinuierlich mit dem Primärsignal bei einem konstanten Pegeldifferential kombiniert, so daß das Sekundärsignal vorzugsweise nicht mehr als 20 dB unter dem Signalpegel des Primärsignals gehalten wird, und in der meist bevorzugten Anwendungsform in etwa zwischen 3 dB - 5 dB unter dem Signalpegel des Primärsignals. Dies verhindert, daß das Sekundärsignal für den Hörer bewußt hörbar wird. Das entstehende kombinierte Signal kann dann durch einen oder mehrere Wiedergabegeräte geschickt werden, so wie Lautsprecher 40 oder Vorverstärker 50 für Tonbandgeräte (nicht abgebildet), um die entstehende subliminale Tonaufnahme zu machen.

Wie oben besprochen, hat die entstehende subliminale Tonaufnahme, in der bevorzugten Anwendungsform, einen Kanal mit vorwärts maskierter Sprache und einen Kanal mit rückwärts maskierter, bzw. Metakontrastsprache. Jeder Kanal hat vorzugsweise drei separate Stimmen - männlich, weiblich und Kinderstimme - die die Nachricht kanonenartig mit voller Echo/Hall Effektierung wiederholen. Wie beschrieben werden wird, wird dies dadurch erreicht, daß verschiedene Apparaturen, wie die in Figur 1a - 1k und Figur 2 gezeigten, parallel verwendet werden.

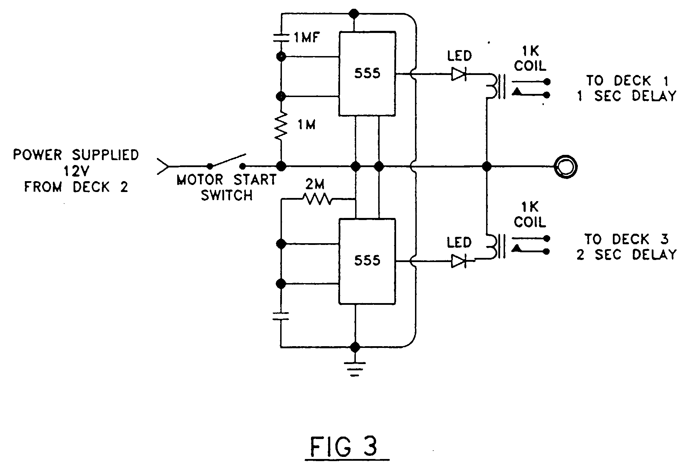

Drei separate Doppelkassettendecks, alle von dem in den Figuren 1a - 1k dargestellten Typ, und als Deck #1, Deck #2 und Deck #3 bezeichnet, werden elektronisch untereinander verbunden. Eines der Decks, z.B. Deck #2, ist mit einem Zeitverzögerungsschaltkreis ausgerüstet, der in Abbildung 3 dargestellt ist (außerdem bei 30 in Figur 1g). Sowohl Deck #1, Deck #2, als auch Deck #3 sind mit zwei voraufgenommennen Bändern bestückt, eines im Band-1 Wiedergabekopf und eines im Band-2 Wiedergabekopf, die die Sprache enthalten, die in den Primärträger eingefügt wird. Sowohl Deck #1, als auch die Decks #2 und #3 haben einen umgedrehten Tonkopf (vorzugsweise Band-2 Wiedergabekopf), so daß eines der Sprachbänder im rückwärts maskierten, bzw. Metakontrastmodus eingefügt wird.

Zusätzlich zu den drei separaten Doppelkassettendecks werden zwei automatische Spurenmischer vom in Figur 2 dargestellten Typ verwendet, und als linker Mischer und rechter Mischer bezeichnet, da jeder Mischer das gesamte Ausgangssignal des linken Kanals und das gesamte Ausgangssignal des rechten Kanals regelt, respektive von jedem einzelnen Kassettendeck.

In Betrieb, nachdem alle sechs voraufgenommenen Bänder in die entsprechenden Wiedergabeköpfe geladen sind, aktiviert der Benutzer das System durch Betätigung des Zeitverzögerungsschalters 30 an Deck #2. Deck #2 wird als erstes aktiviert und arbeitet wie oben beschrieben. Die Betätigung des Zeitverzögerungsschalters aktiviert auch die in Figur 3 dargestellte Zeitverzögerungsschaltung. Diese Schaltung schickt, nach einer Verzögerung von einer Sekunde, Strom von der Stromversorung an Deck #1, und dann nach einer Verzögerung einer weiteren Sekkunde, Strom von der Stromversorgung an Deck #3. Diese Zeitverzögerung hat zum Ergebnis, daß die Sprache von Deck #1, Deck # 2 und Deck #3 kanonartig und mit voller Echo/Hall Effektierung in das Primärsignal eingefügt wird - wie das, was man hören würde wenn man sich Sprache kanonartig gesprochen oder gesungen anhören würde.

Die Ausgangssignale von Deck #1, Deck #2 und Deck #3 werden zu einem der beiden automatischen Spurenmischer gesandt - alle drei linken Ausgangskanäle werden an den linken Mischer gesendet und alle drei rechten Ausgangskanäle werden an den rechten Mischer gesendet. Diese drei kombinierten Signale, die den kanonartigen Effekt ergeben, sind das, was als Sekundärsignal in den Primärträger eingefügt wird, wie es in Figur 2 dargestellt ist. Nachdem der automatische Spurenmischer das Sekundärsignal in den Primärträger einfügt, wobei der angemessene Dezibelabstand (vorzugweise nicht mehr als 20 dB unter dem Pegel des Primärsignals und im günstigsten Fall zwischen 3 dB und 5 dB unter dem Pegel des Primärsignals), wird das entstehende kombinierte Signal auf die jeweils vorgesehene Spur (links oder rechts) der entstehenden subliminalen Tonaufnahme aufgenommen. Dadurch wird das Primärsignal in Stereo aufgenommen und bietet eine angenehme Aufnahme, die leicht anzuhören ist.

Figur 4 zeigt schematisch die Schaltung für die dreifarbige LED-Anzeige, die in dem System der vorliegenden Erfindung verwendet wird. Jeweils eine dreifarbige LED-Anzeige ist an jedem der variablen Widerstände 22 angebracht, die in Figur 1a gezeigt werden. Der Benutzer beabachtet den vom Wiedergabekopf kommenden Signalpegel und justiert den Signalpegel manuell, um ihn auf den gewünschten Dezibelabstand zu den anderen Signalpegeln einzustellen, die von den anderen Wiedergabeköpfen kommen. In der bevorzugten Anwendung sollten die Pegel der einzelnen Signale, die von den jeweiligen Wiedergabeköpfen kommen, im allgemeinen um nicht mehr als ein Dezibel variieren. Um nicht numerische Anzeigen beobachten zu müssen, leuchtet die dreifarbige LED-Anzeige grün, wenn der entsprechende Wiedergabekopf innerhalb eines Dezibel vom erwünschten Referenzpegel liegt. Wenn der Pegel mehr als ein Dezibel unter den erwünschten Referenzpegel fällt, leuchtet die LED rot auf, um den Benutzer zu warnen, daß der Signalpegel zu niedrig ist, damit eine manuelle Korrektur vorgenommen werden kann. Genauso, wenn der Signalpegel mehr als ein Dezibel über den erwünschten Referenzpegel ansteigt, leuchtet die LED gelb, um den Benutzer zu warnen, daß der Pegel zu hoch ist, und eine manuelle Korrektur vorgenommen werden kann. In der bevorzugten Anwendungsform mit Deck #1, Deck #2 und Deck #3, sind zwölf dreifarbige LED-Anzeigeschaltkreise nötig, wie in Figur 4 schematisch dargestellt, da jedes Deck vier Wiedergabeköpfe hat.

Figur 5 zeigt schematisch die Stromversorgung, die in dem System der vorliegenden Erfindung verwendet wird. In der bevorzugten Anwendungsform werden alle drei Decks, zwei Mischer und zwölf LED-Anzeigeschaltungen von einem 120V AC Transformator getrieben, der einen 25V Center Top hat. Der Transformator und der dazugehörige Schaltkreis, der in Figur 5 dargestellt ist, liefert jedem Deck und den begleitenden, peripheren Schaltkreisen, die in anderen Abbildungen gezeigt werden, eine 12V Stromversorgung.

Während die Erfindung im Hinblick auf einzelne ihrer spezifischen Anwendungsformen erklärt worden ist, sollen diese Anwendungsformen eher veranschaulichend als einschränkend betrachtet werden. Es können verschiedene Modifikationen und Zusätze gemacht werden, die für die Fachleute ersichtlich sein werden. Dementsprechend soll die Erfindung durch die vorangegangene Beschreibung nicht eingegrenzt, sondern vielmehr durch die folgenden Patentansprüchen definiert werden.

## Patentansprüche

1. Verfahren zur Herstellung von subliminalen Tonaufnahmen, das folgende Schritte umfaßt:
a) die Selektion eines Klanges, der als Primärträger verwendet wird,
b) die Selektion der Sprache, die unterhalb des Primärträgers als subliminale Nachricht einzufügen ist,
c) die Aufnahme von Sprache in vorwärts abgespielter Form auf einem ersten Kanal und die Aufnahme von Sprache in rückwärts abgespielter Form auf einem zweiten Kanal,
d) die Kombination der Sprache auf dem ersten Kanal mit der Sprache auf dem zweiten Kanal zur Erzeugung der subliminalen Nachricht,
e) das Mischen des Primärträgerklanges mit der Sprache der subliminalen Nachricht, derart, daß gewährleistet ist, daß der Schalldruckpegel der subliminalen Nachricht innerhalb eines konstanten Dezibelabstandes unterhalb des Primärträgerklanges liegt,
f) die Aufnahme der Mischung aus Primärträgerklang und Sprache der subliminalen Nachricht, wobei eine subliminale Tonaufnahme entsteht.

2. Verfahren nach Anspruch 1 oder wenigstens einem der nachfolgenden Ansprüche, **dadurch gekennzeichnet, daß** der konstante Dezibelabstand generell nicht mehr als 20 dB, vorzugsweise nicht mehr als ca. 3-5 dB, beträgt.

3. Verfahren nach wenigstens einem der vorhergehenden oder nachfolgenden Ansprüche, **dadurch gekennzeichnet, daß** zumindest eine Sprache, insbesondere die durch Vorwärts-Aufnahme vorwärts maskierte Sprache, vorzugsweise die vorwärts maskierte und die durch Rückwärts-Aufnahme rückwärts maskierte Sprache, der subliminalen Nachricht eine kanonartige Nachricht ist bzw. sind.

4. Verfahren nach wenigstens einem der vorhergehenden oder nachfolgenden Ansprüche, **dadurch gekennzeichnet, daß** die vorzugsweise kanonartige, subliminale Nachricht ein Zyklus mit drei Wiederholungen ist.

5. Verfahren nach wenigstens einem der vorhergehenden oder nachfolgenden Ansprüche, **dadurch gekennzeichnet, daß** jede subliminale Nachricht, vorzugsweise in Form von dreimal wiederholten Zyklen, eine männliche, ein weibliche und eine Kinderstimme enthält.

6. Verfahren nach einem der vorhergehenden oder nachfolgenden Ansprüche, **dadurch gekennzeichnet, daß** zumindest eine Maskierung, insbesondere die durch Vorwärtsaufnahmen erzeugbare Vorwärtsmaskierung vorzugsweise beide Maskierungen, nämlich die Vorwärts-Aufnahme und die Rückwärtsaufnahme, in voller Echo/Hall-Effektierung durchgeführt ist bzw.sind.

7. Verfahren nach einem der vorhergehenden oder nachfolgenden Ansprüche, **dadurch gekennzeichnet, daß** zumindest eine Nachricht, vorzugsweise die durch Vorwärts-Aufnahme vorwärts und durch Rückwärts-Aufnahme rückwärts maskierte Nachricht, verschiedene Sprachen beinhalten.

8. Verfahren nach wenigstens einem der vorhergehenden Ansprüche 1 bis 6 und wenigstens einem der nachfolgenden Ansprüche, **dadurch gekennzeichnet, daß** wenigstens eine Nachricht, vorzugsweise die durch Vorwärts-Aufnahme vorwärts und durch Rückwärts-Aufnahme rückwärts maskierte Nachricht, dieselbe Sprache beinhalten.

9. Vorrichtung zur Durchführung des Verfahrens nach wenigstens einem der vorhergehenden Ansprüche, das folgendes umfaßt:
a) Hilfsmittel zum Abspielen eines Klanges, der als Primärträger verwendet werden soll,
b) Hilfsmittel zum Abspielen der Sprache, die unterhalb des Primärträgers als subliminale Nachricht eingefügt werden soll,
c) Hilfsmittel zur Aufnahme von Sprache in vorwärts abgespielter Form auf einem ersten Kanal und zur Aufnahme von Sprache in rückwärts abgespielter Form auf einem zweiten Kanal,
d) Mittel zur Kombination der Sprache auf dem ersten Kanal mit der Sprache auf dem zweiten Kanal zur Erzeugung der subliminalen Nachricht,
e)Hilfsmittel zum Mischen des Primärträgerklanges mit der Sprache der subliminalen Nachricht, derart, daß gewährleistet ist, daß der Schalldruckpegel der Sprache der subliminalen Nachricht innerhalb eines konstanten Dezibelbereiches unterhalb des Lautstärkepegels des Primärträgerklanges liegt,
f) Hilfsmittel zur Aufnahme der Mischung aus Primärträgerklang und Sprache der subliminalen Nachricht, wobei eine subliminale Tonaufnahme erstellt wird.

10. Vorrichtung nach Anspruch 9, **dadurch gekennzeichnet, daß** der konstante Dezibelbereich im allgemeinen nicht mehr als 20 dB, vorzugsweise ungefähr 3-5 dB beträgt.

11. Vorrichtung nach einem der vorhergehenden Ansprüche 9 oder 10, **gekennzeichnet durch** folgende Schritte: a)Einrichtungen zum Abspielen der Sprache der subliminalen Nachricht im Vorwärtsmodus und b)Einrichtungen zum Abspielen der Sprache der subliminalen Nachricht im Rückwärtsmodus.

## Claims

1. Method of producing subliminal sound recordings, which includes the following steps:
a) the selection of a sound which is used as the primary carrier;
b) the selection of the language which is to be inserted beneath the primary carrier as the subliminal message;
c) the recording of language in its forwardly played form on a first channel and the recording of language in its backwardly played form on a second channel;
d) the combination of the language on the first channel with the language on the second channel to produce the subliminal message:
e) the mixing of the primary carrier sound with the language of the subliminal message, so that it is ensured that the sound pressure level of the subliminal message lies within a constant decibel range beneath the primary carrier sound; and
f) the recording of the mixture formed from the primary carrier sound and language of the subliminal message, a subliminal sound recording being produced.

2. Method according to claim 1 or at least one of the subsequent claims, characterised in that the constant decibel range is generally no more than 20 dB, preferably no more than approx. 3-5 dB.

3. Method according to at least one of the preceding or subsequent claims, characterised in that at least one language, more especially the language masked forwardly by the forward recording, preferably the forwardly masked language and the language masked backwardly by the backward recording, of the subliminal message is or are a cannon-like message .

4. Method according to at least one of the preceding or subsequent claims, characterised in that the preferably cannon-like subliminal message is a cycle having three repetitions.

5. Method according to at least one of the preceding or subsequent claims, characterised in that each subliminal message, preferably in the form of thrice-repeated cycles, contains a man's voice, a woman's voice and a child's voice.

6. Method according to one of the preceding or subsequent claims, characterised in that at least one masking, more especially the forward masking which can be produced by forward recordings, preferably both maskings, namely the forward recording and the backward recording, is or are carried out with a full echo-chamber effect.

7. Method according to one of the preceding or subsequent claims, characterised in that at least one message, preferably the message masked forwardly by a forward recording and masked backwardly by a backward recording, contains various languages.

8. Method according to at least one of the preceding claims 1 to 6 and at least one of the subsequent claims, characterised in that at least one message, preferably the message masked forwardly by a forward recording and masked backwardly by a backward recording, contains the same language.

9. Apparatus for accomplishing the method according to at least one of the preceding claims, which includes the following:
a) auxiliary means for playing a sound which is to be used as the primary carrier;
b) auxiliary means for playing the language which is to be inserted beneath the primary carrier as the subliminal message;
c) auxiliary means for recording language played in its forwardly played form on a first channel and for recording language played in its backwardly played form on a second channel;
d) means for combining the language on the first channel with the language on the second channel to produce the subliminal message;
e) auxiliary means for mixing the primary carrier sound with the language of the subliminal message, so that it is ensured that the sound pressure level of the language of the subliminal message lies within a constant decibel range beneath the volume level of the primary carrier sound; and
f) auxiliary means for recording the mixture formed from the primary carrier sound and language of the subliminal message, a subliminal sound recording being produced.

10. Apparatus according to claim 9, characterised in that the constant decibel range is generally no more than 20 dB, preferably about 3-5 dB.

11. Apparatus according to one of the preceding claims 9 or 10, characterised by the following steps:
a) arrangements for playing the language of the subliminal message in the forward mode; and
b) arrangements for playing the language of the subliminal message in the backward mode.

## Revendications

1. Procédé pour la réalisation d'enregistrements sonores subliminaux, qui comprend les étapes ci-après:
a) la sélection d'un son qui est utilisé comme support primaire,
b) la sélection de la parole qui doit être insérée en dessous du support primaire à titre de message subliminal,
c) l'enregistrement de la parole sous la forme d'un défilement en marche avant sur un premier canal et l'enregistrement de la parole sous la forme d'un défilement en marche arrière sur un second canal,
d) la combinaison de la parole sur le premier canal avec la combinaison de la parole sur le second canal pour obtenir le message subliminal,
e) le mixage du son de support primaire avec la parole du message subliminal de telle sorte qu'on garantit le fait que le niveau de pression acoustique du message subliminal se situe dans les limites d'un intervalle de décibels constant en dessous du son de support primaire,
f) l'enregistrement du mixage du son de support primaire avec la parole du message subliminal, faisant en sorte que l'on obtient un enregistrement sonore subliminal.

2. Procédé selon la revendication 1 ou selon au moins une des revendications suivantes, caractérisé en ce que l'intervalle de décibels constant n'est généralement pas supérieur à 20 db, de préférence n'est pas supérieur à environ 3-5 db.

3. Procédé selon au moins une des revendications précédentes ou suivantes, caractérisé en ce qu'au moins une parole, en particulier la parole masquée en marche avant grâce à un enregistrement en marche avant, de préférence la parole masquée en marche avant et la parole masquée en marche arrière grâce à un enregistrement en marche arrière, du message subliminal, est, respectivement sont un message en canon.

4. Procédé selon au moins une des revendications précédentes ou suivantes, caractérisé en ce que le message subliminal de préférence en canon est un cycle avec trois répétitions.

5. Procédé selon au moins une des revendications précédentes ou suivantes, caractérisé en ce que chaque message subliminal de préférence sous forme de cycles trois fois répétés contient une voix d'homme, une voix de femme et une voix d'enfant.

6. Procédé selon l'une quelconque des revendications précédentes ou suivantes, caractérisé en ce qu'au moins un masquage, en particulier le masquage en marche avant que l'on obtient à l'aide d'enregistrement en marche avant, de préférence les deux masquages, notamment l'enregistrement en marche avant et l'enregistrement en marche arrière est ou sont réalisés en utilisant complètement l'effet d'écho/Hall.

7. Procédé selon l'une quelconque des revendications précédentes ou suivantes, caractérisé en ce qu'au moins un message, de préférence le message masqué en marche avant à l'aide d'un enregistrement en marche avant et le message masqué en marche arrière à l'aide d'un enregistrement en marche arrière, contient des paroles différentes.

8. Procédé selon au moins une des revendications précédentes 1 à 6 et au moins une des revendications suivantes, caractérisé en ce qu'au moins un message, de préférence le message masqué en marche avant à l'aide d'un enregistrement en marche avant et le message masqué en marche arrière à l'aide d'un enregistrement en marche arrière, contient la même parole.

9. Dispositif pour la mise en oeuvre du procédé selon au moins une des revendications précédentes, qui comprend:
a) un moyen auxiliaire pour l'audition d'un son qui doit être utilisé comme support primaire,
b) un moyen auxiliaire pour l'audition de la parole qui doit être insérée en dessous du support primaire à titre de message subliminal,
c) un moyen auxiliaire pour l'enregistrement de la parole sous forme d'un défilement en marche avant, sur un premier canal, et pour l'enregistrement de la parole sous forme d'un défilement en marche arrière sur un second canal,
d) un moyen pour combiner la parole sur le premier canal avec la parole sur le deuxième canal pour obtenir le message subliminal,
e) un moyen auxiliaire pour mixer le son de support primaire avec la parole du message subliminal de telle sorte qu'on garantit le fait que le niveau de pression acoustique de la parole du message subliminal se situe dans les limites d'un intervalle de décibels constant en dessous du niveau d'isosonie du son de support primaire,
f) un moyen auxiliaire pour l'enregistrement du mixage du son de support primaire avec la parole du message subliminal, faisant en sorte que l'on obtient un enregistrement sonore subliminal.

10. Dispositif selon la revendication 9, caractérisé en ce que l'intervalle de décibels constant n'est généralement pas supérieur à 20 db, de préférence n'est pas supérieur à environ 3-5 db.

11. Dispositif selon l'une quelconque des revendications précédentes 9 ou 10, caractérisé par les éléments ci-après:
a) des mécanismes pour l'audition de la parole du message subliminal dans le mode de marche avant et
b) des mécanismes pour l'audition de la parole du message subliminal dans le mode de marche arrière.
